# EUROPEAN PATENT APPLICATION

(11) **EP 4 678 190 A1**
(43) Date of publication of application: **14.01.2026**
(21) Application number: 24766531.8
(22) Date of filing: 08.03.2024
(51) Int. Cl.: A61K 47/68, A61K 38/07, A61P 35/00, A61P 35/02

(54) **USE OF ANTI-CD20 ANTIBODY-DRUG CONJUGATE IN PREPARATION OF DRUG FOR TREATING MANTLE-CELL LYMPHOMA**

(30) Priority: 09.03.2023 CN 202310223210
(71) Applicant: Zhejiang Teruisi Pharmaceutical Inc., Huzhou, Zhejiang 313000 (CN)
(72) Inventor: HUANG, Kai, Huzhou, Zhejiang 313000 (CN); WANG, Chen, Huzhou, Zhejiang 313000 (CN)
(74) Representative: Haseltine Lake Kempner LLP
(86) International application number: PCT/CN2024/080784
(87) International publication number: WO 2024/183817

(57) **Abstract**

The present disclosure discloses a use of anti-CD20 antibody drug conjugate (ADC) in the preparation of a drug for treating Mantle Cell Lymphoma (MCL). The drug has good clinical efficacy in treating relapsed or refractory mantle cell lymphoma (R/R MCL), is safe and controllable, and meets the currently unmet clinical needs.

## Description

### CROSS-REFERENCE TO RELATED APPLICATIONS

This application claims the benefit of priority to the China Invention Patent Application No. CN202310223210.6 filed on March 9, 2023, the entire contents of the aforementioned application are incorporated herein by reference.

### TECHNICAL FIELD

The present disclosure relates to the field of pharmaceutical technology, and particularly relates to the use of anti-CD20 antibody drug conjugate (ADC) in the preparation of a drug for treating patients with Mantle Cell Lymphoma (MCL).

### BACKGROUND

Mantle Cell lymphoma (MCL) is a rare and invasive subtype of B cell non-Hodgkin lymphoma (NHL). The most common type of MCL originates from mature B cells. It accounts for about 4% and 7%-9% of all malignant lymphomas in the United States and Europe, respectively. In China, MCL accounts for 3.1% of all malignant lymphomas and 4.6% of B-cell lymphomas. The median age of patients is 60 - 70 years old, and about 70% of the patients are male.

MD Anderson Cancer Center in the United States has reported the use of Ibrutinib monotherapy for patients with relapsed or refractory MCL (R/R MCL); the National Comprehensive Cancer Network (NCCN) guidelines recommend Ibrutinib, Zanubrutinib or Acalabrutinib as the preferred second-line treatment for R/R MCL. In the Chinese lymphoma treatment guidelines, no standard treatment regimens for R/R MCL are available. Generally, a regimen that was not used in first-line treatment can be selected. The current second-line recommended regimens include the use of BTK inhibitors (e.g., Ibrutinib, Zanubrutinib or Orelabrutinib) for treatment. However, patients still have a high possibility of recurrence after BTK inhibitor treatment, with a short survival, and the disease is difficult to cure. Available data suggest that although BTK inhibitors (Ibrutinib and Zanubrutinib) improve the prognosis of patients with relapsed MCL and show good safety, the median progression-free survival (PFS) of MCL patients has improved to about 2 years, However, for almost all MCL patients who received treatment with BTK inhibitors, the disease eventually still progressed. In addition, patients who have developed progression after BTK inhibitor treatment have a poor prognosis, with an overall survival (OS) of less than 1 year.

At present, no effective treatment regimen is provided for more than second-line treatment in clinical practice, especially for R/R MCL after BTK inhibitor treatment. Therefore, new treatment regimens are still needed to meet this unmet clinical need and improve the long-term benefit rate and overall survival rate of patients.

### SUMMARY

The purpose of the present disclosure is to provide an anti-CD20 antibody drug conjugate (ADC) thereof in the preparation of a drug for treating Mantle Cell Lymphoma (MCL). The anti-CD20 ADC has good clinical efficacy in treating relapsed or refractory mantle cell lymphoma (R/R MCL) after receiving at least two standard treatments, is safe and controllable, and can meet currently unmet clinical needs.

### A first aspect of the present disclosure provides a use of anti-CD20 ADC in the preparation of a drug for treating MCL.

The anti-CD20 ADC has the following structure: 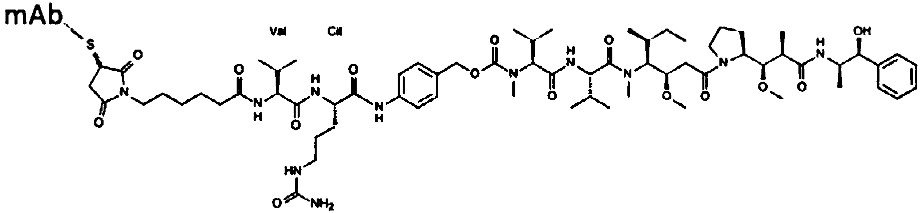 wherein mAb is Rituximab or a biosimilar thereof, and the drug antibody ratio (DAR) is 1 to 8. Preferably, DAR is 4.2 ± 1; further preferably, DAR is 4.2 ± 0.5; and still more preferably, DAR is 4.2 ± 0.3.

According to specific examples of the present disclosure, the anti-CD20 ADC can be "TRS005", and a preparation method thereof is shown in the Patent Application Publication CN108452319A, the entire contents of which are incorporated in the present disclosure.

The drug for treating MCL described in the present disclosure can also be prepared from a pharmaceutical preparation containing the anti-CD20 ADC and a pharmaceutically acceptable carrier or excipient, wherein the pharmaceutical preparation is a liquid preparation or a freeze-dried preparation.

The carrier or excipient is selected from the group consisting of a pH buffer, an osmotic pressure regulator, a lyophilized powder excipient, a protein protecting agent, a solubilizer, and water for injection (WFI).

The pH buffer includes histidine hydrochloride buffer, acetic acid buffer, phosphate buffer, citric acid buffer, or Tris buffer; preferably, the pH buffer is histidine hydrochloride buffer.

The protein protecting agent includes trehalose, sucrose, lactose, or glucose; preferably, the protein protecting agent is sucrose.

The osmotic pressure regulator includes mannitol or sodium chloride; preferably, the osmotic pressure regulator is mannitol.

The solubilizer includes polysorbate 80, polysorbate 20, or poloxamer 188; preferably, the solubilizer is polysorbate 80.

Further, the liquid or freeze-dried preparation includes the above-mentioned anti-CD20 ADC, histidine hydrochloride, trehalose, mannitol, and polysorbate 80. The mantle cell lymphoma (MCL) described in the present disclosure is preferably a relapsed or refractory mantle cell lymphoma (R/R MCL).

Further, the patient with MCL has received at least two standard treatments, which should include at least BTK inhibitor treatment; the BTK inhibitor treatment includes BTK inhibitor monotherapy or BTK inhibitor combined with at least another drug.

The BTK inhibitor includes Ibrutinib, Zanubrutinib, Acalabrutinib, Pirtobrutinib or Orelabrutinib.

The BTK inhibitor monotherapy is treated with one or more of Ibrutinib, Zanubrutinib, Acalabrutinib, Pirtobrutinib or Orelabrutinib.

Further, the treatment with more of Ibrutinib, Zanubrutinib, Acalabrutinib, Pirtobrutinib or Orelabrutinib is the treatment with more of Ibrutinib, Zanubrutinib, Acalabrutinib, Pirtobrutinib or Orelabrutinib in sequence.

The at least another drug includes one or more of Rituximab, Lenalidomide and Venetoclax.

### A second aspect of the present disclosure provides a method for treating MCL patients.

The method comprises administering an effective dose of an anti-CD20 ADC to patients, and the anti-CD20 ADC has the following structure: 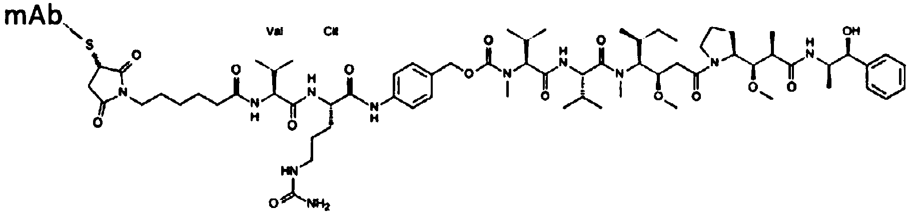 wherein mAb is Rituximab or a biosimilar thereof, and the drug antibody ratio (DAR) is 1 to 8. Preferably, DAR is 4.2 ± 1; further preferably, DAR is 4.2 ± 0.5; and still more preferably, DAR is 4.2 ± 0.3.

According to specific examples of the present disclosure, the anti-CD20 ADC can be "TRS005", and a preparation method thereof is shown in the Patent Application Publication CN108452319A, the entire contents of which are incorporated in the present disclosure.

When administering the anti-CD20 ADC to the MCL patients described in the present disclosure, it can also be administered in the form of a pharmaceutical preparation containing the anti-CD20 ADC and a pharmaceutically acceptable carrier or excipient, and the pharmaceutical preparation is a liquid preparation or a freeze-dried preparation.

The carrier or excipient is selected from the group consisting of a pH buffer, an osmotic pressure regulator, a lyophilized powder excipient, a protein protecting agent, a solubilizer, and water for injection (WFI).

The pH buffer includes histidine hydrochloride buffer, acetic acid buffer, phosphate buffer, citric acid buffer, or Tris buffer; preferably, the pH buffer is histidine hydrochloride buffer.

The protein protecting agent includes trehalose, sucrose, lactose, or glucose; preferably, the protein protecting agent is sucrose.

The osmotic pressure regulator includes mannitol or sodium chloride; preferably, the osmotic pressure regulator is mannitol.

The solubilizer includes polysorbate 80, polysorbate 20, or poloxamer 188; preferably, the solubilizer is polysorbate 80.

Further, the liquid or freeze-dried preparation includes the above-mentioned anti-CD20 ADC, histidine hydrochloride, trehalose, mannitol, and polysorbate 80. The mantle cell lymphoma (MCL) described in the present disclosure is preferably a relapsed or refractory mantle cell lymphoma (R/R MCL).

Further, the patient has received at least two standard treatments, which should include at least BTK inhibitor treatment; the BTK inhibitor treatment includes BTK inhibitor monotherapy or BTK inhibitor combined with at least another drug. The BTK inhibitor includes Ibrutinib, Zanubrutinib, Acalabrutinib, Pirtobrutinib or Orelabrutinib.

The BTK inhibitor monotherapy is treated with one or more of Ibrutinib, Zanubrutinib, Acalabrutinib, Pirtobrutinib or Orelabrutinib.

Further, the treatment with more of Ibrutinib, Zanubrutinib, Acalabrutinib, Pirtobrutinib or Orelabrutinib is specifically treatment with more of Ibrutinib, Zanubrutinib, Acalabrutinib, Pirtobrutinib or Orelabrutinib in sequence.

The at least another drug includes one or more of Rituximab, Lenalidomide and Venetoclax.

The dosage of the anti-CD20 ADC is 0.05 mg/kg to 2.7 mg/kg.

Preferably, the dosage of the anti-CD20 ADC is 0.1 mg/kg to 2.3 mg/kg. Preferably, the dosage of the anti-CD20 ADC is 0.1 mg/kg, 0.5 mg/kg, 1 mg/kg, 1.3 mg/kg, 1.5 mg/kg, 1.8 mg/kg, 2.1 mg/kg, and 2.3 mg/kg.

Preferably, the dosage of the anti-CD20 ADC is 1.5 mg/kg, 1.8 mg/kg and 2.1 mg/kg.

More preferably, the dosage of the anti-CD20 ADC is 1.8 mg/kg.

In the method, the anti-CD20 ADC can be administered once or multiple times. Preferably, the anti-CD20 ADC is administered multiple times, and the administration frequency includes but is not limited to QW, Q2W, Q3W or Q4W. More preferably, the administration frequency is Q3W. Studies have shown that the pharmacokinetic (PK) characteristics of the anti-CD20 ADC of the present disclosure are similar after a single dose and four cycles of administration, and it is almost completely metabolized in the blood after 21 days, supporting the preferred administration frequency of Q3W.

In the method, the administration cycle can be 1-10 treatment cycles, preferably, 6 treatment cycles.

In the method, the anti-CD20 ADC is administered Q3W and continues to be administered until recovery, progression of disease (PD), or death.

In the method, the administration method can be intramuscular, intravenous, intraarterial, intraperitoneal, or subcutaneous administration; preferably, intravenous administration.

It is to be understood that one, some, or all of the properties of the various embodiments described herein may be combined to form other embodiments of the present disclosure. Specific embodiments of the present disclosure are further described in detail as follows.

### DESCRIPTION OF EMBODIMENTS

The present disclosure is described below with reference to specific examples. It will be appreciated by those skilled in the art that these examples are for illustration of the disclosure only and are not intended to limit the scope of the present disclosure in any way.

The experimental methods in the following examples are conventional methods unless otherwise specified. The raw materials, reagent materials, and the like used in the examples described below are commercially available products unless otherwise specified.

### Definition:

"Rituximab" refers to a monoclonal antibody drug targeting CD20 developed by Roche and approved for marketing by the National Medical Products Administration (NMPA) under the trade name of "MabThera". Rituximab is the generic name, and only the innovator drug can be called Rituximab.

"Rituximab biosimilar" refers to the monoclonal antibody drug developed by other companies in addition to the innovator drug of Rituximab, with identical amino acid sequences and comparable physical and chemical properties, efficacy, pharmacokinetics, and safety to the innovator drug.

"Complete Response (CR)" means that the target lesion disappears completely, or all target nodules must be reduced to normal size (short axis < 10 mm), and last for 4 weeks or more.

"Partial Response (PR)" means that the sum of the diameters of all measurable target lesions (long diameter for target lesions and short diameter for target nodules) is reduced by ≥ 50% compared with baseline (in hematological tumors), which lasts for 4 weeks or more.

"Progression of Disease (PD)" means that the minimum value of the sum of all measured diameters of target lesions during the entire tumor treatment process is used as a reference value. The sum of the diameters of target lesions exceeds 20% or more of the reference value, and the absolute value increases by 5 mm or more, or one or more new lesions appear, and CR and PR are not achieved before the size or number of lesions increase.

"Stable Disease (SD)" means that changes in the volume and number of lesions between PR and PD.

"Overall Survival (OS)" refers to the time from randomization (receiving group treatment) to the death or loss to follow-up of the subject for any reason. "Progression-Free Survival (PFS)" refers to the time from randomization to the first tumor recurrence/metastasis, or death of the subject due to any other reason. "Median Progression-Free Survival (mPFS)" refers to the PFS that can be achieved in 50% of evaluable patients.

"Objective Response Rate (ORR)" refers to the sum of the complete response rate (CRR) and the partial response rate (PRR), that is, the proportion of patients who have achieved CR and PR to all evaluable patients.

"Disease control rate (DCR)" refers to the sum of CRR, PRR, and SD rate, that is, the proportion of patients who have achieved CR, PR, and SD to all evaluable patients.

"Refractory" means that PR is not achieved in two treatment cycles, or CR is not achieved in 4 treatment cycles.

"Effective dose" refers to the amount that effectively achieves the desired treatment result at the required dose and time period. The effective dose may vary depending on factors such as the individual's disease state, age, gender, and weight, and the ability of the therapeutic agent or combination of therapeutic agents to induce a desired response in the individual.

"Standard treatment": In the present disclosure, it refers not only to the standard treatment regimen as determined in the conventional clinical sense, but also includes clinically recommended treatment plans, clinical trial treatment plans, and other treatment plans.

Although the present disclosure has been generally described, embodiments of the present disclosure will be further disclosed in the following examples, which should not be construed as limiting the scope of the claims.

### Example 1: Preparation of Anti-CD20 ADC (TRS005)

The preparation method of TRS005 is shown in the Patent Application Publication CN108452319A, the entire contents of which are incorporated in the present disclosure.

The structure of TRS005 is as follows: 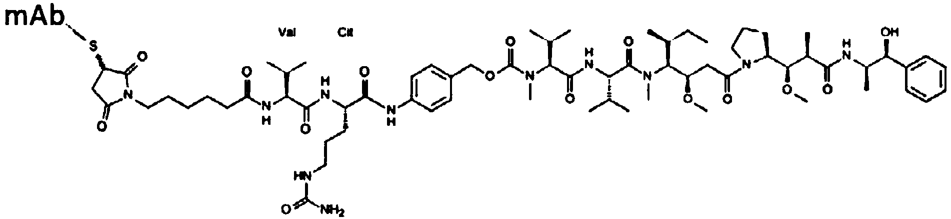 wherein mAb is Rituximab or its biosimilar, and the drug antibody ratio (DAR) is 4.2 ± 0.5.

### Example 2: Study to Evaluate the Safety, Tolerability, Pharmacokinetics, and Effectiveness of TRS005 in Treatment of R/R MCL

The clinical efficacy data of TRS005 are from A Multicenter, Single-arm, Dose-escalating Study to Evaluate the Safety, Tolerability, Pharmacokinetics and Effectiveness of TRS005 in Patients with Relapsed or Refractory CD20-positive B-NHL. The study enrolled 13 MCL subjects from August 24, 2020 to February 23, 2024, and tumor evaluation was completed on 11 subjects (data updated to February 23, 2024).

### (1) Baseline Information of Subjects

All subjects failed at least 2 treatment regimens, and 12 subjects were previously treated with BTK inhibitors. The baseline information of the subjects is shown in Table 1.

**Table 1 Baseline Information of R/R MCL Subjects**

| **Baseline Characteristics** | | **Subjects Enrolled (N=13, %)** |
|---|---|---|
| **Age (Median)** | | 61 |
| | Range | 29-71 |
| | ≥ 60 | 7 (53.8%) |

| **Gender** | | |
|---|---|---|
| | Male | 12 (92.3%) |
| | Female | 1 (7.7%) |

| **ECOG Score** | | |
|---|---|---|
| | 0 | 0 (0.0%) |
| | 1 | 13 (100.0%) |

| **Clinical Phase** | | |
|---|---|---|
| | Phase 3 | 2 (15.4%) |
| | Phase 4 | 7 (53.8%) |
| | Unknown | 4 (30.8%) |

| **Prior Line of Treatment** | | |
|---|---|---|
| | 2 lines | 10 (76.9%) |
| | ≥3 lines | 3 (23.1%) |

| **Baseline LDH Level** | | |
|---|---|---|
| | < 250 | 8 (61.5%) |
| | ≥ 250 | 5 (38.5%) |

| **Prior Treatment** | | |
|---|---|---|
| | BTK inhibitor treatment-experienced | 12 (92.3%) |
| | BTK inhibitor treatment-naive | 1 (7.7%) |

### (2) Administration Regimen

The dosage in this study is 1.5 mg/kg and 1.8 mg/kg, once every 3 weeks (Q3W, i.e., three weeks as one treatment cycle), for a total of 6 treatment cycles. Subjects who have completed the 6 administration cycles can continue to receive donated drug therapy if they have clinical benefits as assessed by the investigator until recovery, progression of disease (PD), or death.

### (3) Efficacy Data

As of February 23, 2024, a total of 13 subjects and 11 subjects in the two dose groups who have completed at least three tumor evaluations after administration were enrolled. The overall efficacy of R/R MCL was ORR 63.6% (7/11) and that of 1.8 mg/kg dose group was 55.6% (5/9). The data are shown in Table 2.

**Table 2 Efficacy Data of R/R MCL Subjects in Different Dose Groups**

| **Efficacy Data** | **1.5 mg/kg (N=2), %** | **1.8 mg/kg (N=9), %** | **Total (N=11)** |
|---|---|---|---|
| CR | 0 | 2 | 2 |
| PR | 2 | 3 | 5 |
| SD | 0 | 2 | 2 |
| PD | 0 | 2 | 2 |
| **ORR** | 100.0% | 55.6% | 63.6% |
| **CRR** | 0.0% | 22.2% | 18.2% |

| | | | |
|---|---|---|---|
| Abbreviations: CR: Complete Response; PR: Partial Response; SD: Stable Disease; PD: Progression of Disease ORR: Objective Response Rate; DCR: Disease Control Rate | | | |

A total of 10 subjects had previously received BTK inhibitors, including Zanubrutinib and Orelabrutinib. The study showed that the overall efficacy for this group of patients was ORR 60% (6/10), including 2 cases of CR and 4 cases of PR.

The data are shown in Table 3.

**Table 3 Efficacy Data of R/R MCL Subjects who Previously Received BTK Inhibitors in Different Dose Groups**

| **Efficacy Data** | **1.5 mg/kg (N=1), %** | **1.8 mg/kg (N=9), %** | **Total (N=10)** |
|---|---|---|---|
| CR | 0 | 2 | 2 |
| PR | 1 | 3 | 4 |
| SD | 0 | 2 | 2 |
| PD | 0 | 2 | 2 |
| **ORR** | 100.0% | 55.6% | 60% |
| **CRR** | 100.0% | 22.2% | 20% |

| | | | |
|---|---|---|---|
| Abbreviations: CR: Complete Response; PR: Partial Response; SD: Stable Disease; PD: Progression of Disease ORR: Objective Response Rate; CRR: Disease Control Rate | | | |

### (4) Safety Data

As of February 02, 2024, a total of 12 (92.31%) of the 13 subjects with mantle cell subtypes enrolled in this study experienced 217 adverse events (AEs), all of which were TEAEs, of which 187 were judged by the investigator to be related to the investigational drug TRS005 (definitely related, probably related or possibly related). A total of 9 (69.23%) subjects experienced 36 ≥ Grade 3 TEAEs, of which 8 (61.54%) subjects had a total of 29 AEs judged to be related to the investigational drug. A total of 1 (7.69%) subject had a dose reduction due to TEAEs, which was judged to be drug-related; a total of 3 (23.08%) subjects had medication interruption due to TEAEs, all of which were judged to be related to the investigational drug.

Among the 13 subjects with mantle cell subtypes enrolled, a total of 6 (46.15%) subjects experienced 12 serious adverse events (SAEs), of which 10 in 4 subjects were judged to be related to the investigational drug. Most of the SAEs have recovered or are recovering.

Based on the above clinical safety data, it is comprehensively assessed that the investigational drug TRS005 has good safety in subjects with mantle cell subtypes.

In summary, in the Phase 1 clinical study conducted, for subjects who were previously treated with BTK inhibitors, the overall objective response rate (ORR) of the investigational drug TRS005 was 60%, indicating that TRS005 can still achieve excellent clinical benefits in the R/R MCL patient population after previous treatment with BTK inhibitors, and that TRS005 has excellent efficacy and controllable safety in this indication population, with very good drug potential. In addition, another patient who was not previously treated with BTK inhibitors also achieved PR, suggesting that TRS005 can achieve good efficacy and drug potential in R/R MCL after previous treatment with or without BTK inhibitors. Given that there is still a lack of reliable clinical treatment regimens for R/R MCL after BTK inhibitor treatment, TRS005 is expected to meet the clinical needs of this indication population.

The above description of embodiments is not intended to limit the present disclosure. Those skilled in the art may make various changes or modifications according to the present disclosure, and such changes or modifications are intended to fall within the scope of the appended claims if they do not depart from the spirit of the disclosure.

## Claims

1. A use of anti-CD20 antibody drug conjugate (ADC) in the preparation of a drug for treating Mantle Cell Lymphoma (MCL), wherein the anti-CD20 ADC has the following structure: 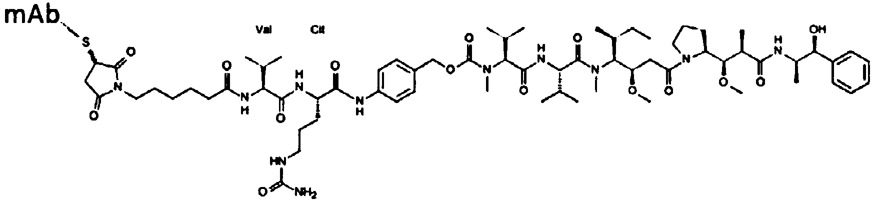 wherein mAb is Rituximab or a biosimilar thereof, and drug antibody ratio (DAR) is 1 to 8; preferably, DAR is 4.2 ± 1; further preferably, DAR is 4.2 ± 0.5; and still more preferably, DAR is 4.2 ± 0.3.

2. The use according to any one of claim 1, wherein the MCL is a relapsed or refractory mantle cell lymphoma (R/R MCL).

3. The use according to claim 2, wherein a patient with the MCL has received at least two standard treatments, which includes at least BTK inhibitor treatment.

4. The use according to claim 3, wherein the BTK inhibitor treatment includes BTK inhibitor monotherapy or BTK inhibitor combined with at least another drug.

5. The use according to any one of claims 3-4, wherein the BTK inhibitor includes Ibrutinib, Zanubrutinib, Acalabrutinib, Pirtobrutinib or Orelabrutinib.

6. The use according to any one of claims 4-5, wherein the BTK inhibitor monotherapy is treated with one or more of Ibrutinib, Zanubrutinib, Acalabrutinib, Pirtobrutinib or Orelabrutinib.

7. The use according to claim 6, wherein the treatment with more of Ibrutinib, Zanubrutinib, Acalabrutinib, Pirtobrutinib or Orelabrutinib is the treatment with more of Ibrutinib, Zanubrutinib, Acalabrutinib, Pirtobrutinib or Orelabrutinib in sequence.

8. The use according to any one of claims 4-5, wherein at least another drug includes one or more of Rituximab, Lenalidomide and Venetoclax.

9. A method for treating MCL patient, wherein the method comprises administering an effective dose of an anti-CD20 ADC to the patient, and the anti-CD20 ADC has the following structure: 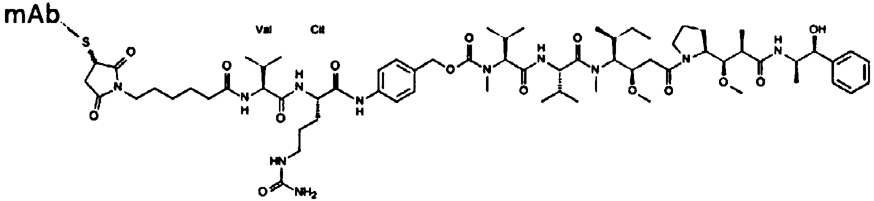 wherein mAb is Rituximab or a biosimilar thereof, and the DAR is 1 to 8; preferably, DAR is 4.2 ± 1; further preferably, DAR is 4.2 ± 0.5; and still more preferably, DAR is 4.2 ± 0.3.

10. The method according to claim 9, wherein the MCL is preferably a relapsed or refractory mantle cell lymphoma (R/R MCL).

11. The method according to claim 10, wherein the patient has received at least two standard treatments, which includes at least BTK inhibitor treatment.

12. The method according to claim 11, wherein the BTK inhibitor treatment includes BTK inhibitor monotherapy or BTK inhibitor combined with at least another drug.

13. The method according to any one of claims 11-12, wherein the BTK inhibitor includes Ibrutinib, Zanubrutinib, Acalabrutinib, Pirtobrutinib or Orelabrutinib.

14. The method according to any one of claims 12-13, wherein the BTK inhibitor monotherapy is treated with one or more of Ibrutinib, Zanubrutinib, Acalabrutinib, Pirtobrutinib or Orelabrutinib.

15. The method according to any one of claim 14, wherein the treatment with more of Ibrutinib, Zanubrutinib, Acalabrutinib, Pirtobrutinib or Orelabrutinib is specifically treatment with more of Ibrutinib, Zanubrutinib, Acalabrutinib, Pirtobrutinib or Orelabrutinib in sequence.

16. The method according to any one of claims 12-13, wherein at least another drug includes one or more of Rituximab, Lenalidomide and Venetoclax.

17. The method according to any one of claims 9-16, wherein the dosage of the anti-CD20 ADC is 0.05 mg/kg to 2.7 mg/kg; preferably, the dosage of the anti-CD20 ADC is 0.1 mg/kg to 2.3 mg/kg; preferably, the dosage of the anti-CD20 ADC is 0.1 mg/kg, 0.5 mg/kg, 1 mg/kg, 1.3 mg/kg, 1.5 mg/kg, 1.8 mg/kg, 2.1 mg/kg, and 2.3 mg/kg; preferably, the dosage of the anti-CD20 ADC is 1.5 mg/kg, 1.8 mg/kg, and 2.1 mg/kg; and most preferably, the dosage of the anti-CD20 ADC is 1.8 mg/kg.

18. The method according to claim 17, wherein the anti-CD20 ADC can be administered once or multiple times; preferably, the anti-CD20 ADC is administered multiple times, and the administration frequency includes but is not limited to QW, Q2W, Q3W, or Q4W; and more preferably, the administration frequency is Q3W.

19. The method according to claim 18, wherein the anti-CD20 ADC is administered Q3W and continues to be administered until recovery, progression of disease (PD), or death.
